Europäisches Patentamt

European Patent Office

Office européen des brevets

(19)

(11) Numéro de publication : **0 230 184 B1**

(12) **FASCICULE DE BREVET EUROPEEN**

(45) Date de publication du fascicule du brevet :
23.01.91 Bulletin 91/04

(51) Int. Cl.⁵ : **F16L 35/00**

(21) Numéro de dépôt : **86402790.9**

(22) Date de dépôt : **12.12.86**

(54) Tuyau à raccord de sécurité, notamment pour usages médicaux.

(30) Priorité : 13.12.85 FR 8518494
17.10.86 FR 8614467

(43) Date de publication de la demande :
29.07.87 Bulletin 87/31

(45) Mention de la délivrance du brevet :
23.01.91 Bulletin 91/04

(84) Etats contractants désignés :
AT BE CH DE ES FR GB GR IT LI LU NL SE

(56) Documents cités :
DE-A- 2 941 020
FR-A- 2 321 089
GB-A- 872 908
GB-A- 2 131 118

(73) Titulaire : DUFFOUR ET IGON S.A. (D.I.)
Rue de l'Oasis
F-31000 Toulouse (FR)

(72) Inventeur : Vigneau, Jean-Louis
Montegut Plantaurel
F-09120 Varilhes (FR)
Inventeur : Trenque, Pierre
1, résidence du Parc
F-31520 Ramonville St Agne (FR)

(74) Mandataire : Lemoine, Robert et al
Cabinet Malémont 42, Avenue du Président
Wilson
F-75116 Paris (FR)

EP 0 230 184 B1

## Description

La présente invention concerne un tuyau à raccord de sécurité, notamment pour le transport de gaz à usages médicaux, dans lequel le raccord se compose d'un porte-griffes monté en rotation autour d'un corps central traversé par un canal et comportant, à l'extérieur du porte-griffes, un embout tubulaire sur lequel une extrémité libre du tuyau est emmanchée et fixée, le corps central portant en outre une rondelle saillante amovible s'appuyant sur une face du porte-griffes, pour l'immobiliser en translation avec un épaulement du corps situé de l'autre côté.

Comme on le sait, ces raccords de sécurité connus portent des griffes d'accouplement dont la forme et la disposition mutuelle sont différentes suivant le type de gaz que doit véhiculer le tuyau qu'ils équipent, si bien qu'ils ne peuvent être connectés qu'à un raccord de configuration complémentaire associé à la source de gaz correspondante.

Les tuyaux munis de tels raccords de sécurité sont essentiellement utilisés dans les établissements de soins où, notamment lors d'une·anesthésie ou d'une réanimation, ils suppriment les risques d'une interversion du branchement entre par exemple les sources d'oxygène et de protoxyde d'azote.

Toutefois, les appareils d'anesthésie ou de réanimation sont équipés d'un grand nombre de ces tuyaux à raccord de sécurité et on est amené à remplacer très fréquemment les tuyaux proprement dits, qui à cet effet sont fixés sur leur raccord à l'aide d'un collier de serrage amovible. Or ce remplacement est bien souvent effectué par le personnel technique ou médical qui, en remettant en place les tuyaux neufs sur les raccords, peut inverser deux tuyaux et donc fausser les branchements ultérieurs, avec les plus graves conséquences pour les patients.

Pour éliminer ce risque, on a eu l'idée de solidariser de façon définitive le tuyau au raccord. Dans ce cas toutefois, lors d'un remplacement du tuyau, on ne peut bien entendu pas récupérer le raccord fort coûteux qui, étant d'une durée de vie quasiment illimitée, est encore en parfait état.

La présente invention se propose de remédier à ces inconvénients et, pour ce faire, elle a pour objet un tuyau à raccord de sécurité du type spécifié en préambule qui se caractérise en ce que la rondelle saillante s'appuie sur la face du porte-griffes tournée vers l'embout, et la zone de fixation du tuyau sur le raccord ainsi que les parties voisines de ces deux derniers, sont complètement emprisonnées dans une coquille protectrice monobloc qui se prolonge jusqu'au porte-griffes avec lequel elle est en contact glissant en recouvrant par son extrémité la rondelle, cette coquille protectrice monobloc étant en outre indéformable et immobilisée en translation, de sorte qu'elle ne peut être retirée qu'après avoir été désintégrée et donc sans qu'il soit possible de la remettre en place autour du raccord.

On comprendra aisément que, grâce à ces dispositions, tout accès direct à la zone de fixation entre le tuyau et le raccord, sans destruction de la coquille protectrice, est interdit. Il s'ensuit que dans les établissements de soin, le personnel ne sera pas incité à remplacer lui-même les tuyaux défectueux puisqu'il ne serait pas en mesure de reconstituer la coquille et par conséquent les risques d'erreur évoqués plus haut sont supprimés.

De même, la coquille protectrice en recouvrant complètement la rondelle qui retient le porte-griffes sur le corps du raccord, interdit tout délogement volontaire ou prémédité de cette dernière et donc toute manoeuvre malveillante d'interversion des porte-griffes entre deux raccords différents.

Avantageusement, la coquille protectrice est formée de deux demi-coquilles identiques, solidarisées l'une à l'autre le long d'un plan de joint passant par l'axe central de l'embout tubulaire ; dans le cas où les demi-coquilles sont constituées d'une matière plastique dure, cette solidarisation est réalisée de préférence par un soudage ultrasonique, selon une technique connue en elle-même, notamment d'après GB-A-2 131 118.

La coquille protectrice peut ainsi être cassée et remplacée par un élément de mêmes caractéristiques et qualité par le fabricant, à qui on pourra donc, en toute sécurité, confier l'opération de remplacement du tuyau défectueux. Dans ces conditions, le raccord pourra être récupéré, ce qui réduira considérablement le coût de cette opération.

Selon un premier mode de réalisation du tuyau à raccord de sécurité selon·l'invention dans lequel l'extrémité libre du tuyau, qui est souple, est fixée sur l'embout tubulaire du raccord au moyen d'un collier de serrage amovible qui présente des éléments saillants, la coquille protectrice recouvre les éléments saillants du collier de serrage par des parties creuses en relief qui, au moins dans la direction axiale, épousent sensiblement la forme desdits éléments saillants, pour constituer ainsi l'un des moyens d'immobilisation en translation de la coquille protectrice.

Selon un second mode de réalisation de l'invention, qui sera plus préférentiellement retenu, l'extrémité libre du tuyau, qui est souple est fixée sur l'embout tubulaire du raccord par une ou plusieurs lèvre(s) de compression saillant sur la face intérieure de la coquille protectrice, sur une hauteur suffisante pour pincer le tuyau contre l'embout du raccord.

Avantageusement, la ou les lèvres(s) de compression est ou sont venue(s) de matière avec le reste de la coquille protectrice.

De la sorte, la coquille protectrice, en plus de constituer une sécurité interdisant les branchements erronés, remplit la fonction d'un organe de sertissage du tuyau sur le raccord et rend donc inutile l'utilisation des colliers de serrage traditionnels. La conséquence première en est une grande simplification de l'opération de remplacement des tuyaux usagés de par la suppression des étapes les plus longues d'enlèvement et de remise en place du collier de serrage.

Dans une forme de réalisation préférée, la coquille protectrice porte intérieurement deux lèvres de compression radiales qui sont disposées en vis-à-vis et qui, de préférence, couvrent chacune un peu moins d'une demi-circonférence du tuyau, pour que les efforts de compression se répartissent le plus uniformément possible sur ce dernier.

La coquille protectrice pourra en outre porter une couleur identifiant le fluide que le tuyau à raccord doit véhiculer ou, en variante, dans le cas où il s'agit d'un mélange binaire, les deux demi-coquilles pourront être teintées de couleurs différentes identifiant respectivement les deux composants du mélange.

Deux modes de réalisation de la présente invention vont maintenant être décrits plus en détails, mais uniquement à titre d'exemples non-limitatifs, en référence aux dessins annexés, dans lesquels :

– la figure 1 est une vue en coupe longitudinale d'un raccord de sécurité droit monté sur l'extrémité libre d'un tuyau, avec une coquille de protection conforme à un premier mode de réalisation de l'invention ;

– la figure 2 est une vue en coupe effectuée selon la ligne II-II de la figure 1 ;

– les figures 3 et 4 sont respectivement une vue en coupe longitudinale et une vue en plan d'un raccord de sécurité coudé monté sur l'extrémité libre d'un tuyau, avec un coquille de protection conforme au premier mode de réalisation de l'invention ; et

– les figures 5, 7 et 8 sont des vues analogues respectivement aux figures 1, 3 et 4, mais illustrant une coquille de protection conforme à un second mode de réalisation de l'invention, la figure 6 étant quant à elle une vue en coupe agrandie selon la ligne VI-VI de la figure 5.

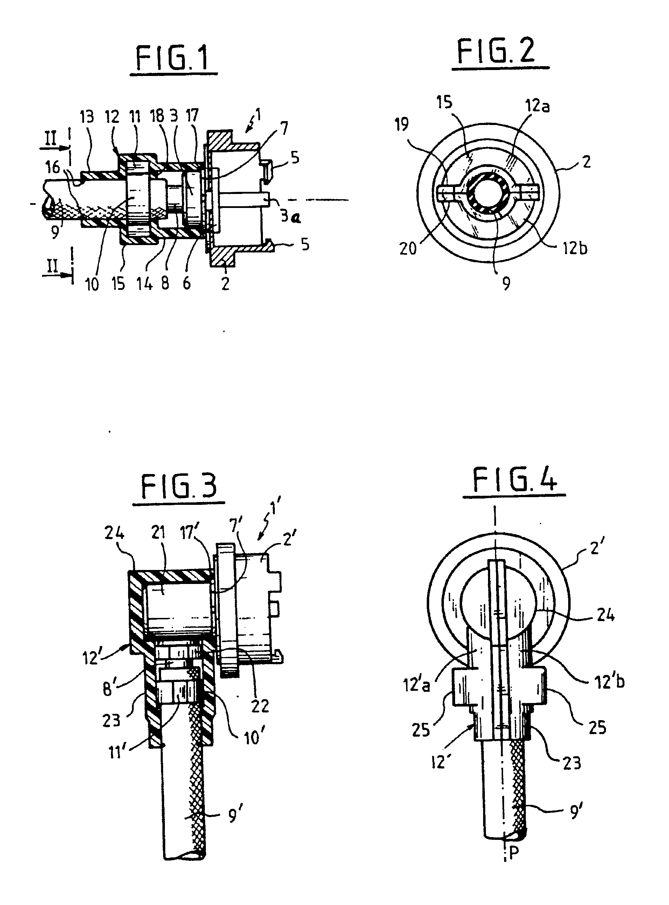

Le raccord de sécurité droit 1, visible sur la figure 1, présente une structure connue en soi et on s'attachera donc à n'en décrire que les caractéristiques essentielles.

Comme on peut le voir, ce raccord 1 comprend un porte-griffes creux monté coaxialement et à rotation libre autour d'un corps cylindrique central 3 percé, tout le long de son axe longitudinal, d'un canal non représenté qui se prolonge à l'intérieur du porte-griffes par un mince conduit de connexion 3a.

Plus précisément, le porte-griffes 2, muni sur la face libre de griffes d'accouplement 5, s'appuie du côté de ces dernières sur un disque 6 venu de matière avec le corps 3 et est immobilisé en translation, de l'autre côté, par une rondelle fendue saillante 7 encliquetée autour de ce dernier.

Le corps central 3 comprend quant à lui, à l'extérieur du porte-griffes 2, un embout coaxial tubulaire 8 à travers lequel le canal du corps se prolonge. L'extrémité libre d'un tuyau souple 9 est sertie sur cet embout 8 au moyen d'un collier de serrage amovible 10 pourvu de deux oreilles latérales saillantes 11.

Selon la caractéristique première de l'invention, une coquille protectrice monobloc 12 emprisonne complètement le collier de serrage 10 en se refermant de part et d'autre sur le tuyau 9, cette coquille 12 étant réalisée en une matière plastique dure et cassable et présentant une paroi pleine.

Comme le montre clairement la figure 1, la coquille 12 comprend un premier et un second tronçons tubulaires d'extrémité 13 et 14, qui sont réunis par un court tronçon annulaire en relief 15.

Plus précisément, le premier tronçon tubulaire 13 présente, au niveau de son extrémité libre, une légère surépaisseur intérieure 16 grâce à laquelle il enserre fermement le tuyau 9 en amont du collier de serrage 10. De son côté, le second tronçon tubulaire 14, qui est d'un diamètre légèrement supérieur à celui du premier, se prolonge jusqu'à la face extérieure 2a du porte-griffes 2, avec laquelle il est en contact glissant, et se termine par une gorge annulaire intérieure 17 par laquelle il recouvre la rondelle fendue 7.

Enfin, le tronçon central en relief 15 délimite une cavité annulaire dans laquelle viennent se loger étroitement les deux oreilles 11 du collier de serrage 10. Au niveau de la jonction entre le tronçon central 15 et le second tronçon tubulaire 14, une lèvre annulaire 18 fait en outre saillie à l'intérieur de la coquille 12 pour venir s'appliquer étroitement tout autour de l'extrémité libre du tuyau 9, située en aval du collier 10.

En se reportant maintenant à la figure 2, on peut voir que la coquille 12 est formée de deux demi-coquilles 12a, 12b qui sont symétriques par rapport à un plan longitudinal P passant par l'axe central X de l'embout 8 et sont solidarisées l'une à l'autre le long de ce plan, de préférence par un soudage ultrasonique. On observera encore que, tout le long de leur plan de joint P, les deux demi-coquilles 12a et 12b portent chacune des nervures de renforcement 19 et 20 extérieures.

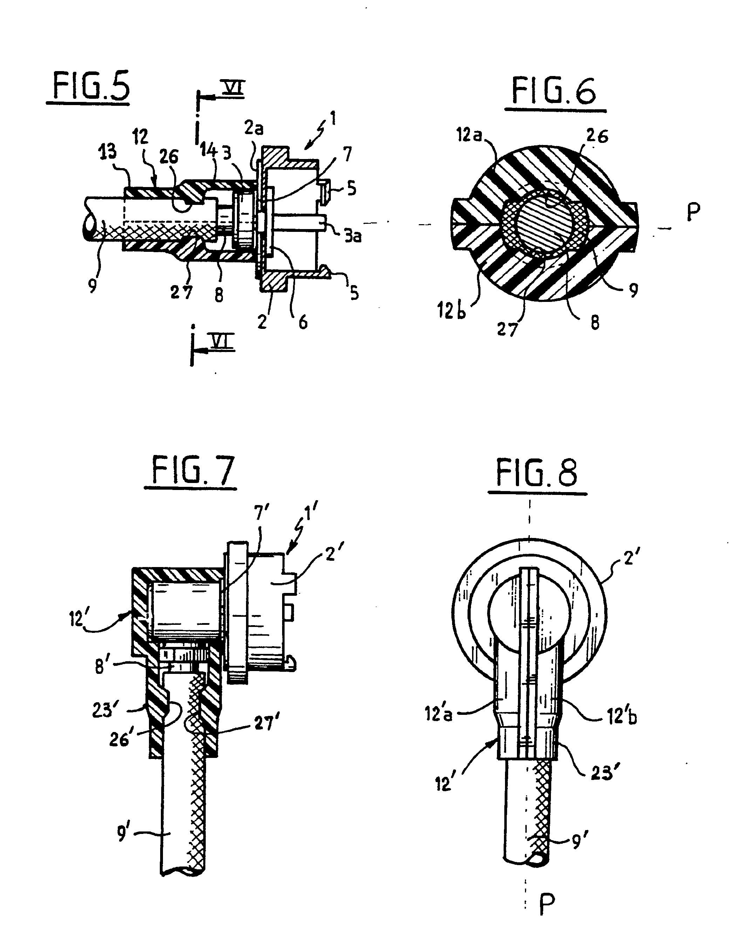

Les figures 3 et 4 représentant un raccord rotatif coudé 1' qui ne se distingue du raccord rotatif droit 1 des figures 1 et 2 que sur le mode de liaison entre le corps et l'embout 8'.

Plus précisément, la partie du corps, saillant à l'extérieur du porte-griffes 2', est constituée par une pièce cylindrique fermée 21 de grande longueur et l'embout tubulaire 8' vient se visser à angle droit dans un perçage taraudé de la paroi latérale de cette pièce cylindrique 21. Une collerette à six pans 22 est d'ailleurs prévue sur l'embout 8' pour faciliter ce vissage par utilisation d'une clé plate.

La coquille 12' présente une forme correspondante définie par un premier tronçon sensiblement tubulaire 23 qui s'étend jusqu'à la pièce cylindrique 21 en enveloppant l'extrémité libre du tuyau 9', le collier

de serrage 10′ et l'embout 8′ et par une calotte de section circulaire 24 qui présente un axe longitudinal perpendiculaire à celui du premier tronçon 23, pour pouvoir envelopper complètement la pièce cylindrique 21, y compris la rondelle fendue 7′, par une gorge annulaire intérieure 17′.

Enfin, les deux demi-coquilles 12′a, 12′b comprennent respectivement deux zones renflées rectangulaires 25 (figure 4) qui sont symétriques par rapport au plan de joint P des demi-coquilles 12a, 12b pour recevoir les oreilles saillantes 11′ du collier de serrage 10′ sans gêner la mise en place de la coquille 12′.

On va maintenant décrire, en référence aux figures 5 à 8, un second mode de réalisation de l'invention. Les raccords représentés sur ces figures étant en tous points identiques à ceux des figures 1 à 4, ils ne seront pas décrits une nouvelle fois et leurs différents éléments portent les mêmes références que sur ces figures. De la même façon, les parties communes aux coquilles protectrices des premier et second modes de réalisation sont désignées par les mêmes références et ne seront pas redécrites.

La seule différence qui distingue la coquille protectrice 12, 12′ du second mode de réalisation de celle du premier tient au fait qu'elle remplit la fonction supplémentaire de sertissage du tuyau 9 ou 9′ sur l'embout tubulaire 8,8′ du raccord 1 ou 1′, si bien que l'on peut se passer d'un organe de fixation classique, tel que le collier de serrage 10 ou 10′ des figures 1 à 4 et que les parties en relief 15, 25 associées de la coquille disparaissent. Pour réaliser ce sertissage, la coquille 12 ou 12′ porte intérieurement deux lèvres ou mâchoires de compression saillantes 26,27 ou 26′,27′, qui s'étendent en vis-à-vis dans un même plan perpendiculaire à l'axe de l'embout 8 ou 8′ et sont d'une hauteur suffisante pour s'enfoncer dans le tuyau souple 9 ou 9′ et le coincer contre l'embout du raccord.

Sur le raccord droit représenté sur la figure 5, les deux lèvres 26, 27 sont formées au niveau de la zone de transition entre les deux tronçons tubulaires 13, 14, de diamètres différents, de la coquille 12, c'est-à-dire là où se trouvaient le tronçon annulaire en relief 15 du premier mode de réalisation de la figure 1. La figure 6 montre en outre que chaque demi-coquille 12a ou 12b porte une lèvre de compression respective. Sur cette même figure, on peut encore voir que les lèvres de compression 26, 27 ont une forme en arc de cercle et couvrent respectivement un peu moins d'une demi-circonférence du tuyau 9, c'est-à-dire environ 140-150°, tout en ayant des positions symétriques par raport au plan de joint P des demi-coquilles 12a et 12b. Comme cela va de soi, les lèvres de compression 26, 27 sont venues de matière avec les demi-coquilles 12a, 12b respectives et seront, pour ce faire, produites directement lors du moulage de ces dernières.

Sur le raccord coudé représenté sur la figure 7, les deux lèvres de compression 26′, 27′ sont formées à l'intérieur du premier tronçon tubulaire 23′de la coquille 12′, là où se trouvaient les deux renflements rectangulaires 25 du premier mode de réalisation. Pour le reste, le dimensionnement et le positionnement mutuel des lèvres de compression ainsi que leur mode de formation sont en tous points identiques à ce qu'ils sont dans la coquille 12 des figures 5 et 6.

Pour être complet, on précisera encore que la coquille 12 ou 12′ du raccord selon l'invention sera colorée d'une teinte indentifiant le gaz destiné à être transporté par le tuyau 9 ou 9′ ou que, dans le cas où ce gaz est un mélange binaire, les deux demi-coquilles 12a, 12b ou 12′a, 12′b qui la constituent seront colorées différemment pour permettre l'identification des deux composants du mélange gazeux.

Comme on l'aura compris, la coquille protectrice 12 ou 12′ des premier et second modes de réalisation de l'invention qui viennent d'être décrits, a pour but d'interdire tout accès direct à la zone de fixation entre le tuyau et le raccord et dès lors d'empêcher un remplacement des tuyaux usagés par le personnel d'un établissement de soins, remplacement qui pourrait être à l'origine d'une interversion de tuyaux entre des raccords associés à des sources de gaz différentes.

Le remplacement des tuyaux sera, confié à un spécialiste, tel que le fabricant qui pour ce faire commencera par casser, à l'aide d'un outil classique, la coquille en matière plastique, retirera éventuellement le collier de serrage (uniquement dans le cas du raccord des figures 1 à 4) pour séparer le tuyau usagé du raccord, puis remettra en place un tuyau neuf sur ce dernier avant de revouvrir convenablement la zone de fixation du tuyau de deux demi-coquilles 12a, 12b ou 12′a, 12′b, préablement moulées et ayant la couleur correspondant à la source de gaz associée au raccord,et de souder finalement l'une à l'autre les deux demi-coquilles qui dès lors formeront une nouvelle coquille protectrice 12, 12′ présentant les même caractéristiques et qualité que la coquille de départ. Si le raccord est équipé d'une coquille 12, 12′ selon les figures 5 à 8, cette opération de remplacement sera grandement simplifée et réduite en durée de par la suppression du démontage et du montage du collier de serrage 10, 10′ du premier mode de réalisation.

## Revendications

1. Tuyau à raccord de sécurité, notamment pour le transport de gaz à usages médicaux, dans lequel le raccord (1 ; 1′) se compose d'un porte-griffes (2 ; 2′) monté en rotation autour d'un corps central (3 ; 3′) traversé par un canal et comportant, à l'extérieur du porte-griffes, un embout tubulaire (8 ; 8′) sur lequel une extrémité libre du tuyau (9 ; 9′) est emmanchée et fixée, le corps central portant en outre une rondelle

saillante amovible (7 ; 7′) s'appuyant sur une face du porte-griffes, pour l'immobiliser en translation avec un épaulement (6) du corps situé de l'autre côté, caractérisé en ce que la rondelle saillante (7 ; 7′) s'appuie sur la face du portegriffes tournée vers l'embout, et la zone de fixation du tuyau (9 ; 9′) sur le raccord (1 : 1′) ainsi que les parties voisines de ces deux derniers, sont complètement emprisonnés dans une coquille protectrice monobloc (12 ; 12′) qui se prolonge jusqu'au porte-griffes (2 ; 2′) avec lequel elle est en contact glissant en recouvrant par son extrémité (17 ; 17′) la rondelle (7 ; 7′), cette coquille protectrice monobloc étant en outre indéformable et immobilisée en translation, de sorte qu'elle ne peut être retirée qu'après avoir été désintégrée et donc sans qu'il soit possible de la remettre en place autour du raccord

2. Tuyau à raccord de sécurité selon la revendication 1, caractérisé en ce que la coquille (12, 12′) est teintée d'une ou de plusieurs couleurs identifiant le fluide destiné à être véhiculé par le tuyau ou respectivement, dans le cas d'un mélange, ses différents composants.

3. Tuyau à raccord de sécurité selon la revendication 1 ou 2, caractérisé en ce que la coquille protectrice (12 ; 12′) est formée de deux demi-coquilles identiques (12a, 12b ; 12′a, 12′b) solidarisées l'une à l'autre le long d'un plan de joint passant par l'axe central de l'embout tubulaire (8 ; 8′).

4. Tuyau à raccord de sécurité selon la revendication 3, caractérisé en ce que les deux demi-coquilles (12a, 12b ; 12′a, 12′b) sont en une matière plastique et sont solidarisées l'une à l'autre par un soudage ultrasonique.

5. Tuyau à raccord de sécurité selon la revendication 3 ou 4, destiné à véhiculer un fluide formé d'un mélange de deux composants, caractérisé en ce que les deux demi-coquilles (12a, 12b) sont teintées de deux couleurs différentes identifiant respectivement les composants du mélange.

6. Tuyau à raccord de sécurité selon l'une quelconque des revendications 1 à 5, dans lequel l'extrémité libre du tuyau (9 ; 9′), qui est souple, est fixée sur l'embout tubulaire (8 ; 8′) du raccord (1 ; 1′) au moyen d'un collier de serrage amovible (10 ; 10′) qui présente des éléments saillants (11 ; 11′), caractérisé en ce que la coquille protectrice (12 ; 12′) recouvre les éléments saillants (11 ; 11′) du collier de serrage (10 ; 10′) par des parties creuses en relief (15 ; 25) qui, au moins dans la direction axiale, épousent sensiblement la forme desdits éléments saillants, pour constituer le moyen d'immobilisation en translation de la coquille.

7. Tuyau à raccord de sécurité selon l'une quelconque des revendications 1 à 5, caractérisé en ce que l'extrémité libre du tuyau (9 ; 9′), qui est souple, est fixée sur l'embout tubulaire (8 ; 8′) du raccord (1 ; 1′) par une ou plusieurs lèvre(s) de compression (26, 27 ; 26′, 27′) saillant sur la face intérieure de la coquille protectrice (12 ; 12′), sur une hauteur suffisante pour pincer le tuyau contre l'embout du raccord.

8. Tuyau à raccord de sécurité selon la revendication 7, caractérisé en ce que la ou les lèvre(s) de compression (26, 27 ; 26′, 27′) est ou sont venue(s) de matière avec le reste de la coquille protectrice (12; 12′).

9. Tuyau à raccord de sécurité selon la revendication 7 ou 8, caractérisé en ce que la coquille protectrice (12 ; 12′) porte intérieurement deux lèvres de compression radiales (26, 27 ; 26′, 27′) qui sont disposées en vis-à-vis et couvrent chacune un peu moins d'une demi-circonférence du tuyau (9 ; 9′).

## Ansprüche

1. Rohr mit Sicherheitskupplung, insbesondere für die Gasleitung bei medizinischen Anwendungen, wobei die Kupplung (1 ; 1′) aus einem Klauenträger (2 ; 2′) besteht, der um einen mittleren Körper (3 ; 3′) drehbar gelagert ist, der von einem Kanal durchdrungen ist und außerhalb des Klauenträgers einen Rohransatz (8 ; 8′) trägt, auf den ein freies Ende des Rohrs (9 ; 9′) aufgebracht und befestigt ist, wobei der mittlere Körper außerdem eine abnehmbare vorspringende Scheibe (7 ; 7′) trägt, die sich auf eine Seite des Klauenträgers abstützt, um diesen mittels eines Absatzes (6) des Körpers auf der anderen Seite gegen eine Verschiebung festzulegen, **dadurch gekennzeichnet,** daß die vorspringende Scheibe (7; 7′) sich auf die dem Ansatz gewandte Seite des Klauenträgers abstützt, und der befestigungsbereich des Rohrs (9 ; 9′) an der Kupplung (1 ; 1′) sowie die benachbarten Teile dieser zwei Letzteren vollständig in einer einstückigen Schutzumhüllung (12 ; 12′) eingeschlossen sind, die sich bis zum Klauenträger (2 ; 2′) ausdehnt, mit dem sie in Gleitberührung steht, indem sie mit ihrem Ende (17 ; 17′) die Scheibe überdeckt, wobei diese einstückige Schutzumhüllung außerdem unverformbar und verschiebungsfest ist, so daß sie nur dann entfernt werden kann, wenn sie zerkleinert worden ist und es daher nicht möglich ist, sie um die Kupplung wieder aufzustellen.

2. Rohr mit Sicherheitskupplung nach Anspruch 1, **dadurch gekennzeichnet,** daß die Umhüllung (12; 12′) mit einer oder mehreren Farben gefärbt ist, die das durch das Rohr zu leitende Fluid oder bei einer Mischung deren einzelne bestandteile kennzeichnet.

3. Rohr mit Sicherheitskupplung nach Anspruch 1 oder 2, **dadurch gekennzeichnet,** daß die Schutzumhüllung (12 ; 12′) aus zwei identischen Halbschalen (12a, 12b ; 12′a, 12′b) gebildet ist, die miteinander längs einer Verbindungsebene fest verbunden sind, die durch die Mittelachse des Rohransatzes (8 ; 8′) hindurchgeht.

4. Rohr mit Sicherheitskupplung nach Anspruch 3, **dadurch gekennzeichnet,** daß die zwei Halbscha-

len (12a, 12b ; 12′a, 12′b) aus Kunststoff bestehen und durch Ultraschallschweißen miteinander fest verbunden sind.

5. Rohr mit Sicherheitskupplung nach Anspruch 3 oder 4, welches zum Leiten eines von einem Zweikomponenten-Gemisch gebildeten Fluids bestimmt ist, **dadurch gekennzeichnet,** daß die zwei Halbschalen (12a, 12b) mit unterschiedlichen Farben gefärbt sind, die die jeweiligen bestandteile der Mischung kennzeichnen.

6. Rohr mit Sicherheitskupplung nach irgendeinem der Ansprüche 1 bis 5, bei der das freie Ende des Rohrs (9 ; 9′), welches nachgiebig ist, auf dem Rohransatz (8 ; 8′) der Kupplung (1 ; 1′) mittels eines entfernbaren Klemmbandes (10 ; 10′) befestigt ist, welches zwei vorspringende Elemente (11 ; 11′) aufweist, **dadurch gekennzeichnet,** daß die Schutzumhüllung (12 ; 12′) die vorspringenden Elemente (11 ; 11′) des Klemmbandes (10 ; 10′) mit erhabenen, hohlen bereichen (15 ; 25) überdeckt, die zumindest in der axialen Richtung im wesentlichen der Form der genannten vorspringenden Elemente folgen, zur Bildung des Mittels, das die Umhüllung gegen eine Verschiebung festlegt.

7. Rohr mit Sicherheitskupplung nach irgendeinem der Ansprüche 1 bis 5, **dadurch gekennzeichnet,** daß das freie Ende des Rohrs (9 ; 9′), welches nachgiebig ist, auf dem Rohransatz (8 ; 8′) der Kupplung (1 ; 1′) mittels einer oder mehrerer Zusammendrücklippe(n) (26, 27 ; 26′, 27′) befestigt ist, die auf der Innenseite der Schutzumhüllung (12 ; 12′) mit einer ausreichenden Höhe hervorsteht bzw. hervorstehen, um das Rohr gegen den Anschlußansatz zu klemmen.

8. Rohr mit Sicherheitskupplung nach Anspruch 7, **dadurch gekennzeichnet,** daß die Zusammendrücklippe(n) (26, 27 ; 26′, 27′) mit der übrigen Schutzumhüllung (12 ; 12′) aus einem Material hergestellt ist bzw. sind.

9. Rohr mit Sicherheitskupplung nach Anspruch 7 ou 8, **dadurch gekennzeichnet,** daß die Schutzumhüllung (12 ; 12′) innen zwei radiale Zusammendrücklippen (26, 26 ; 26′, 27′) trägt, die einander gegenüberliegend angeordnet sind und jeweils etwas weniger als einen halben Umfang des Rohrs (9 ; 9′) überdecken.

## Claims

1. A pipe with a safety coupling, particularly for conveying gases for medical uses, wherein the coupling (1 ; 1′) comprises a claw holding member (2 ; 2′) rotatably mounted around a central body (3 ; 3′) which is passed through by a channel and including, externally of the claw holding member, a tubular tip (8 ; 8′) having a free end of the pipe (9 ; 9′) fitted and attached thereon, the central body further bearing a removable projecting washer (7 ; 7′) seating on a face of the claw holding member to secure it translationally with a body shoulder (6) located on the other side, characterized in that the projecting washer (7 ; 7′) is seating on the face of the claw holding member which is directed towards the tip, the region for fastening the pipe (9 ; 9′) on the coupling (1 ; 1′) as well as the parts adjacent to the two latter being entirely confined in a one-piece protective shell (12 ; 12′) extending to the claw holding member (2 ; 2′) with which it is in sliding engagement by covering the washer (7 ; 7′) with its end (17 ; 17′), said one-piece protective shell further being non deformable and translationally secured, so that it is removable only after being disintegrated and hence without it being possible to replace it around the coupling.

2. The pipe with a safety coupling according to claim 1, characterized in that the shell (12 ; 12′) is coloured with one or several colours identifying the fluid to be conveyed through the pipe or, in the case of a mixture, the various components thereof, respectively.

3. The pipe with a safety coupling according to claim 1 or 2, characterized in that the protective shell (12 ; 12′) is formed of two identical half-shells (12a, 12b ; 12′a, 12′b) secured together along a joint plane which crosses the central axis of the tubular tip (8 ; 8′).

4. The pipe with a safety coupling according to claim 3, characterized in that the two half-shells (12a, 12b ; 12′a, 12′b) are made of plastic and are secured together by ultrasonic welding.

5. The pipe with a safety coupling according to claim 3 or 4, for conveying a fluid formed of a mixture of two components, characterized in that the two half-shells (12a, 12b) are coloured with two different colours respectively identifying the components of the mixture.

6. The pipe with a safety coupling according to any of claims 1 to 5, wherein the free end of the pipe (9 ; 9′) which is flexible, is attached on the tubular tip (8 ; 8′) of the coupling (1 ; 1′) by means of a removable clamping collar (10 ; 10′) provided with projections (11 ; 11′), characterized in that the protective shell (12 ; 12′) covers the projections (11 ; 11′) of the clamping collar (10 ; 10′) by raised hollow parts (15 ; 25) substantially conforming to said projections, at least in the axial direction, to form the translationally securing means of the shell.

7. The pipe with a safety coupling according to any of claims 1 to 5, characterized in that the free end of the pipe (9 ; 9′) which is flexible, is secured on the tubular tip (8 ; 8′) of the coupling (1 ; 1′) by a least one compression lip (26, 27 ; 26′, 27′) projecting high enough from the internal face of the protective shell (12 ; 12′) to pinch the pipe against the tip of the coupling.

8. The pipe with the safety coupling according to claim 7, characterized in that the at least one compression lip (26, 27 ; 26′, 27′) is integrally formed with the remaining protective shell (12 ; 12').

9. The pipe with a safety coupling according to claim 7 or 8, characterized in that the protective shell (12 ; 12') is provided internally with two facing radial compression lips (26, 27 ; 26', 27') each covering slightly less than half a circumference of the pipe (9 ; 9').

7

FIG.1

FIG.2

FIG.3

FIG.4

## FIG.5

## FIG.6

## FIG.7

## FIG.8